# EUROPEAN PATENT SPECIFICATION

(11) **EP 3 035 776 B1**
(45) Date of publication and mention of the grant of the patent: **15.02.2017**
(21) Application number: 14198364.3
(22) Date of filing: 16.12.2014
(51) Int. Cl.: H05H 7/12, A61N 5/00, G21K 1/00

(54) **Energy degrader**
Energiedegrader
Dispositif de dégradation d'énergie

(43) Date of publication of application: 22.06.2016
(73) Proprietor: ION BEAM APPLICATIONS S.A., 1348 Louvain-la-Neuve (BE)
(72) Inventor: Claereboudt, Yves, 1457 Nil-Saint-Vincent (BE)
(74) Representative: De Groote, Christophe

(56) References cited:
- EP-A1- 1 371 390
- EP-A1- 1 541 194
- WO-A2-2009/063353
- US-A- 5 039 867

## Description

### Field of the invention

The invention relates to the field of charged particle accelerators, such as proton or carbon ion accelerators for example, and more particularly to an energy degrader for attenuating the energy of a charged particle beam produced by such a particle accelerator.

### Description of prior art

Certain applications involving the use of beams of charged particles require the energy of these particles to be varied. This is for example the case in particle therapy applications, where the energy of the charged particles determines the depth of penetration of these particles into a body to be treated by such therapy. Some charged particle accelerators, such as synchrotrons for instance, are adapted to vary the energy of the particle beam which they produce, but it may nevertheless be desirable to further vary the energy after the particles have been extracted from the synchrotron. Other particle accelerators, such as cyclotrons for instance, are not themselves adapted to vary the energy of the particle beam which they produce, and therefore require an additional device to vary this energy.

Devices for varying the energy of a particle beam extracted from a particle accelerator are often called energy degraders. An energy degrader comprises therefore one or more blocks of matter which are placed across the path of the particle beam after its extraction from the particle accelerator. According to a well-known principle, any particle passing through such a block of matter undergoes a decrease in its energy by an amount which is, for particles of a given type, a function of the intrinsic characteristics of the material passed through and of its thickness.

A known energy degrader is for example disclosed in US6433336. It comprises a single block of matter which has the shape of a helicoidal staircase. The particle beam enters the degrader perpendicularly to a step of the staircase and exits the degrader at an opposite side, which attenuates the energy of the beam according to the thickness of the degrader at said step. After having rotated the staircase by a given angle around its axis, the beam will enter the degrader perpendicularly to another step, which will attenuate the energy of the beam by a different amount according to the thickness of the degrader at said other step. The energy attenuation can thus be varied by changing the angular position of the degrader with respect to the particle beam.

Another known energy degrader is for example disclosed in US2014091238. It comprises two wedge-shaped blocks of matter which are transversally movable into the beam path. The energy attenuation is varied by changing the transversal position of the blocks with respect to the particle beam.

Another known energy degrader is for example disclosed in EP1371390. It comprises two pairs of wedge-shaped blocks of matter which are transversally movable into the beam path. The basic energy attenuation is modified by changing the transversal position of the pair of largest blocks with respect to the particle beam. The pair of smallest blocks is used to adjust/modulate the energy attenuation during irradiation, for example to adapt it to breathing of the patient.

A drawback of these known energy degraders is that the attenuator blocks which are used to set the basic energy attenuation are heavy and that it is therefore difficult to move them quickly and/or with high accuracy with respect to the particle beam. Some recent applications require however to be able to change the energy of the particle beam very quickly, such as in a few tens of milliseconds for instance, and/or with high positional accuracy.

This is for example the case with particle therapy systems, where a target, such as a tumour for example, is to be irradiated layer by layer with the particle beam, these layers being at different depths into the body of the patient. In such cases, it is desirable to be able to change the basic energy of the particle beam very quickly and/or very accurately when the system passes from one layer to another layer.

### Summary of the invention

It is an object of the invention to address the drawbacks of the known energy degraders. It is a particular object of the invention to provide an energy degrader which is adapted to vary the basic energy of a particle beam more quickly and/or with higher accuracy than the known degraders.

A typical beam energy upstream (i.e. at an input) of an energy degrader according to the invention is in the MeV range, such as in the range of 150 MeV to 300 MeV for example, and a typical desired basic beam energy downstream (i.e. at an output) of the energy degrader according to the invention is also in the MeV range, such as in the range of 50 MeV to 230 MeV for an input energy of 230 MeV for example.

The invention is defined by the independent claims. The dependent claims define advantageous embodiments.

According to the invention, there is provided an energy degrader for attenuating the energy of a charged particle beam, such as a beam of protons or of carbon ions for example, said energy degrader comprising:
- a first energy attenuation member (A) adapted to attenuate the energy of charged particles crossing said first attenuation member,
- a second energy attenuation member (B) adapted to attenuate the energy of charged particles crossing said second attenuation member, and
- a drive unit operably connected to the first and to the second energy attenuation members and configured for moving the first and/or the second energy attenuation members across the charged particle beam,
wherein:
- the mass (m_{A}) of the first energy attenuation member (A) is smaller than the mass (m_{B}) of the second energy attenuation member (B),
- the drive unit is configured for moving simultaneously the first energy attenuation member (A) at a first speed (VA1) and the second energy attenuation member (B) at a second speed (VB1) across the charged particle beam during a first movement,
- the shapes of the first and the second energy attenuation members (A, B) are such that a total energy attenuation of the particle beam remains constant during the first movement,
- the drive unit is configured for moving the first energy attenuation member (A) at a third speed (VA2) across the charged particle beam during a second movement, and
- the average third speed (VA2) over the second movement is larger than the average second speed (VB1) over the first movement.

By being configured for moving simultaneously the lighter energy attenuation member (A) at the first speed (VA1) and the heavier energy attenuation member (B) at the second speed (VB1) across the charged particle beam during the first movement, yet keeping a total energy attenuation of the particle beam constant during the first movement, the drive unit may reposition the lighter energy attenuation member (A) with respect to the particle beam so that it is ready for the second movement, yet without having to move the heavier energy attenuation member (B) quickly.. In a particle therapy system for instance, this first movement may for instance be performed in the course of the irradiation of a given layer of the target.

Furthermore, by being configured for moving the lighter energy attenuation member (A) at the higher speed (VA2) across the charged particle beam during the second movement, one can indeed vary the total energy attenuation more quickly and with higher accuracy than with the conventional energy degraders. In a particle therapy system for instance, this second movement and the accompanying variation of the energy of the particle beam may for instance be performed during a change of layer of the target to be irradiated.

Preferably, the mass (m_{A}) of the first energy attenuation member is smaller than 0.5 times the mass (m_{B}) of the second energy attenuation member.

More preferably, the mass (m_{A}) of the first energy attenuation member is smaller than 0.1 times the mass (m_{B}) of the second energy attenuation member Even more preferably, the mass (m_{A}) of the first energy attenuation member is smaller than 0.02 times the mass (m_{B}) of the second energy attenuation member.

Preferably, the first energy attenuation member is made of the same material as the second energy attenuation member.

Preferably, the average third speed (VA2) over the second movement is larger than two times the average second speed (VB1) over the first movement. More preferably, the average third speed (VA2) over the second movement is larger than five times the average second speed (VB1) over the first movement. Even more preferably, the average third speed (VA2) over the second movement is larger than ten times the average second speed (VB1) over the first movement.

According to the invention, there is preferably provided an energy degrader for attenuating the energy of a charged particle beam, said energy degrader comprising:
- a first energy attenuation member (A) adapted to attenuate the energy of charged particles crossing said first attenuation member and having the shape of a wedge presenting a first beam entry face (A1) and an opposed first beam exit face (A2),
- a second energy attenuation member (B) adapted to attenuate the energy of charged particles crossing said second attenuation member and having the shape of a wedge presenting a second beam entry face (B1) and an opposed second beam exit face (B2),
- said first and second beam entry faces as well as said first and second beam exit faces being flat faces,
- a drive unit operably connected to the first and to the second energy attenuation members and configured for moving the first and/or the second energy attenuation members across the charged particle beam,
wherein:
- the mass (m_{A}) of the first energy attenuation member (A) is smaller than the mass (m_{B}) of the second energy attenuation member (B),
- the drive unit is configured for moving simultaneously the first energy attenuation member (A) at a first speed (VA1) and the second energy attenuation member (B) at a second speed (VB1) across the charged particle beam during a first translational movement,
- the shapes of the first and the second energy attenuation members (A, B) are such that a total energy attenuation of the particle beam remains constant during the first movement,
- the drive unit is configured for moving the first energy attenuation member (A) at a third speed (VA2) across the charged particle beam during a second translational movement, and
- the average third speed (VA2) over the second movement is larger than the average second speed (VB1) over the first movement.

Such a preferred configuration allows indeed for much more flexibility in the variation of the attenuation of the energy of the particle beam, compared to cases where the energy attenuation members have other shapes and/or compared with cases where the movements of the energy attenuation members are not translational movements.

In such a preferred configuration, the first beam entry face (A1) is preferably parallel to the second beam exit face (B2), the first beam exit face (A2) is preferably parallel to the second beam entry face (B1), and the drive unit is preferably configured in such a way that, during the first movement, the instantaneous first speed (VA1) is equal to the instantaneous second speed (VB1). This allows for the particle beam to enter and exit the energy degrader perpendicularly to the first entry face and to the last exit face, thereby reducing unwanted distortions on the particle beam.

According to the invention, there is also provided a particle therapy system comprising a particle accelerator configured for producing a charged particle beam, and comprising an energy degrader according to the invention for attenuating the energy of the charged particle beam output by the particle accelerator. The particle accelerator is preferably a fixed-energy accelerator, more preferably a cyclotron, for example a synchrocyclotron.

### Short description of the drawings

These and further aspects of the invention will be explained in greater detail by way of example and with reference to the accompanying drawings in which:
- Fig.1: schematically shows an energy degrader according to the invention;
- Fig.2: schematically shows an energy degrader according to the invention, with an exemplary drive unit;
- Fig.3: schematically shows an energy degrader according to the invention, with a preferred drive unit;
- Fig.4: schematically shows a preferred energy degrader according to the invention;
- Figs. 5a, 5b, 5c: schematically show a cross section of the energy degrader of Fig. 4 at various stages of a movement of its components;
- Fig.6: schematically shows a more preferred energy degrader according to the invention;
- Fig.7: schematically shows an even more preferred energy degrader according to the invention;
- Figs. 8a, 8b, 8c, 8d: schematically show cross sections of the energy degrader of Fig. 7 and according to different arrangements of its components and of their movements;
- Fig.9: schematically shows a variant of an energy degrader according to
- an: an example;
- Fig.10: schematically shows another variant of an energy degrader according to the invention;
- Fig.11: schematically shows a part of a particle therapy system comprising a particle accelerator and an energy degrader according to the invention.

The drawings of the figures are neither drawn to scale nor proportioned. Generally, similar or identical components are denoted by the same reference numerals in the figures.

### Detailed description of embodiments of the invention

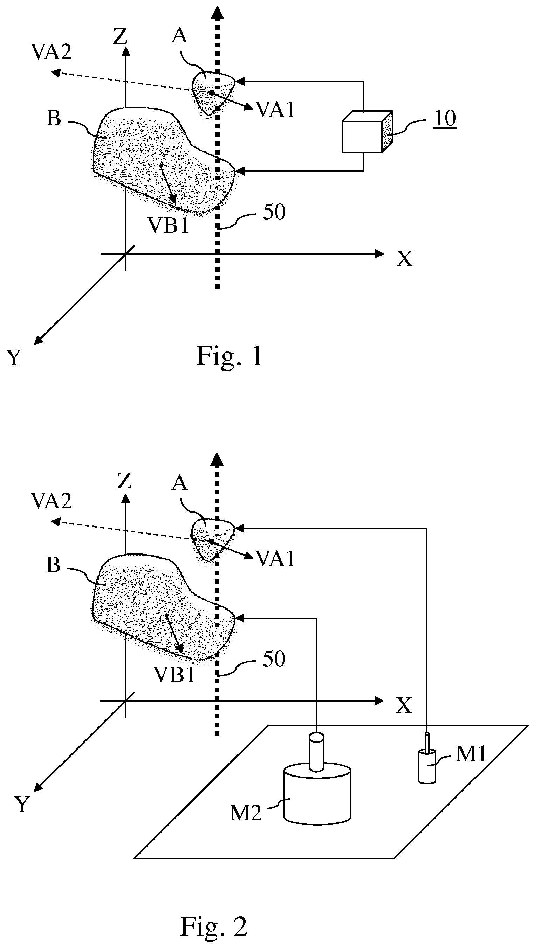

Fig.1 schematically shows a 3D view of an energy degrader according to the invention. The energy degrader comprises a first energy attenuation member (A) adapted to attenuate the energy of charged particles crossing said first attenuation member and a second energy attenuation member (B) adapted to attenuate the energy of charged particles crossing said second attenuation member. As is well known in the art, these attenuation members are for example blocks of solid matter such as blocks of Beryllium or of Carbon graphite for example. Preferably, the first energy attenuation member (A) is made of the same material as the second energy attenuation member (B). Specific to the invention is that the mass (m_{A}) of the first energy attenuation member (A) is smaller than the mass (m_{B}) of the second energy attenuation member (B). Preferably, m_{A} is smaller than 0.5 times the m_{B}. More preferably, m_{A} is smaller than 0.1 times the m_{B}. Even more preferably, m_{A} is smaller than 0.02 times the m_{B}. Exemplary masses will be given hereafter.

A charged particle beam (50) crossing both the first and the second energy attenuation members is shown on Fig.1. In operation, the energy degrader will thus attenuate the energy of the particle beam (50) following to its crossing of the both the first and the second energy attenuation members, as shown on Fig.1. A total attenuation of the energy of the particle beam (50) may be estimated as the sum of the energy attenuations provided by the first and the second energy attenuation members along the path of the particle beam (50). The energy degrader further comprises a drive unit (10) which is operably connected to the first and to the second energy attenuation member for moving the first and/or the second energy attenuation members across the charged particle beam (50). Such drive units are also well known in the art. The drive unit (10) is configured for moving simultaneously the first energy attenuation member (A) at a first speed (VA1) and the second energy attenuation member (B) at a second speed (VB1) across the charged particle beam (50) during a first movement. Depending on the shapes of the energy attenuation members and on their respective speeds, the energy attenuation will vary or not vary in the course of this first movement. Preferred examples of speeds and shapes resulting in the total energy attenuation remaining constant during this first movement will be given hereafter.

The drive unit (10) is further configured for moving the first energy attenuation member (A) at a third speed (VA2) across the charged particle beam (50) during a second movement. In the course of this second movement, the second energy attenuation member (B) may or may not move. However, the shapes and the positions of the first and second energy attenuation members shall preferably be chosen in such a way that, when in operation, the particle beam (50) crosses both energy attenuation members during the first movement as well as during the second movement.

Specific to the invention is that the average third speed (VA2) over the second movement is larger than the average second speed (VB1) over the first movement. Preferably, the average third speed (VA2) over the second movement is larger than two times the average second speed (VB1) over the first movement. More preferably, the average third speed (VA2) over the second movement is larger than five times the average second speed (VB1) over the first movement. Even more preferably, the average third speed (VA2) over the second movement is larger than ten times the average second speed (VB1) over the first movement. For a particle therapy application, it is for example advantageous and found to be feasible that VB1 is in the range of 0.02 m/s to 0.2 m/s (for example 10cm/5s to 10cm/500ms), preferably in the range of 0.05 m/s to 0.1 m/s (for example 10cm/2s to 10cm/1s), and that VA2 is in the range of 0.2 m/s to 100 m/s (for example 10cm/500 ms to 10cm/1ms), preferably in the range of 1 m/s to 2 m/s (for example 10cm/100ms to 10cm/50ms).

In any and all of these cases, the drive unit (10) is preferably configured in such a way that, at any instant in the course of the first movement, the instantaneous first speed (VA1) is equal to the instantaneous second speed (VB1) (both speeds being considered as vector quantities in this case). This allows indeed for the total energy attenuation to depend solely or substantially on the shapes of the first and second energy attenuation members, which simplifies the design of the system.

Fig.2 schematically shows an energy degrader according to the invention, with an exemplary drive unit (10). In this example, a first motor (M1) is operably connected to move the first energy attenuation member (A) at the first speed (VA1) during the first movement and at the third speed (VA2) during the second movement, with respect to a stationary part - such as a chassis for example - or to the particle beam (50). A second motor (M2) is operably connected to move the second energy attenuation member (B) at the second speed (VB1) during the first movement, with respect to the stationary part or to the particle beam (50).

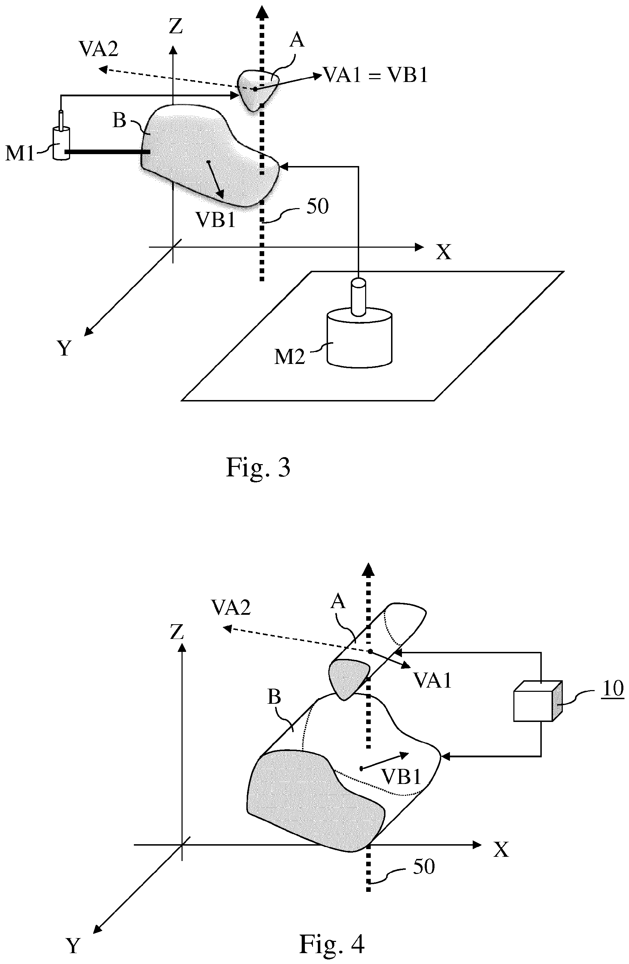

Fig.3 schematically shows a preferred energy degrader according to the invention, with a preferred drive unit (10). In this example, a second motor (M2) is operably connected to move the second energy attenuation member (B) at the second speed (VB1) during the first movement, with respect to a stationary part - such as a chassis for example - or to the particle beam (50). A first motor (M1), whose stator is rigidly connected to the second energy attenuation member (B), is operably connected to move the first energy attenuation member (A) at the first speed (VA1) during the first movement and at the third speed (VA2) during the second movement, with respect to the second attenuation member. Hence, by operating the second motor (M2) while not operating or while blocking the first motor (M1), both the first and the second energy attenuation members will move simultaneously and at the same speed with respect to the chassis or to the particle beam (50) during the first movement, and, by operating the first motor (M1), the first energy attenuation member (A) will move with respect to the chassis or to the particle beam (50) during the second movement.

In the examples given hereafter and wherein the energy attenuation members are to be moved into translation, and in case the motor is a rotating type motor such as an electric motor for instance, one will of course make use of an intermediary transmission (not shown) in order to transform the rotational movement of the motor(s) into a translational movement applied to the energy attenuation members (A, B). In the examples given hereafter and wherein the energy attenuation members are to be moved into rotation, and in case the motor is a rotating type motor, one may also use an intermediary transmission (not shown) in order to adapt the speed and/or the torque applied to the energy attenuation members (A, B).

Fig.4 schematically shows a 3D view of a preferred energy degrader according to the invention in an XYZ referential. As can be seen on this figure, the first and the second energy attenuation members each have a cylindrical shape (a shape obtained by moving a straight line parallel to itself, in this example parallel to the Y axis). In this case, the drive unit (10) is preferably configured to move the first and the second energy attenuation members in translation in a plane parallel to the XZ plane (vectors VA1 and VA2 are parallel to XZ).

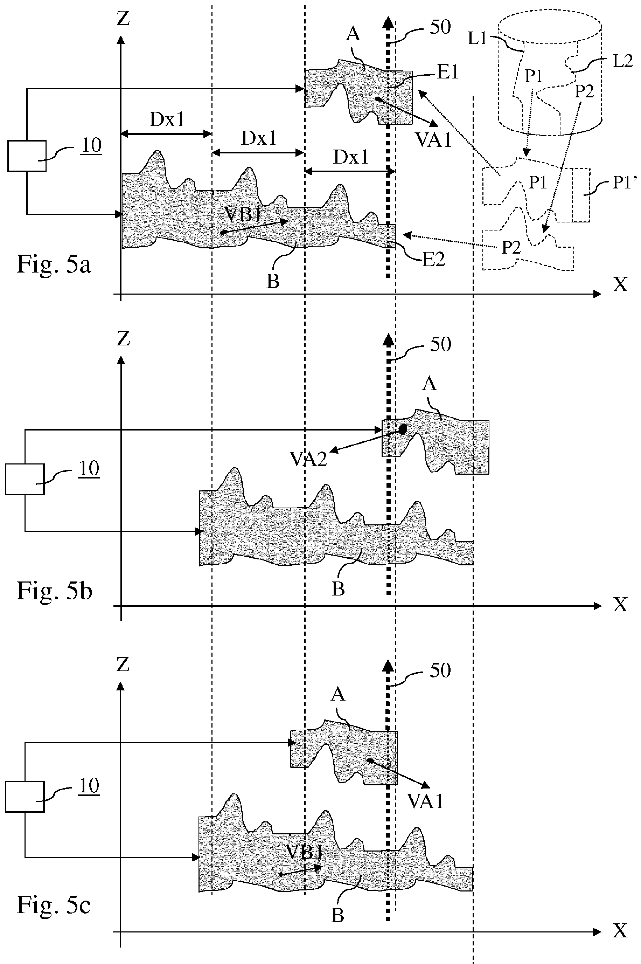

Figs. 5a, 5b, 5c schematically show cross sections of an exemplary and preferred energy degrader according to Fig. 4, at various stages of a movement of the first and the second energy attenuation members , the cross sections being taken according to a plane parallel to the XZ plane. In these figures, an imaginary particle beam (50) is show whose path is parallel to the Z axis.
Such preferred energy degrader is particularly useful for particle therapy, as will become clearer hereafter.
The cross sections are special here. They are obtained by dividing the surface of an imaginary cylinder along two freely chosen lines (L1, L2), yielding thus two imaginary parts (P1, P2), and by deploying these two parts so that they become flat, as shown at the right side of Fig. 5a in dashed lines. A flat rectangular appendix (P1') is moreover added to the right side of the first part (P1). P1 and P1' together represent the cross section of the first energy attenuation member (A).

The second part (P2) represents the cross section of the rightmost part of the second energy attenuation member (B), namely whose width equals to Dx1. The two other parts of the second energy attenuation member (B) which are at the left of this rightmost part each have a width which equals to Dx1. They are obtained by increasing the height of the second part (P2) without changing the top and bottom profiles of the second part and by respectively aligning the corresponding top and bottom profiles.
The way the drive unit (10) moves the first and the second energy attenuation members will now be described in more detail.

Fig. 5a shows the positions of the first and the second energy attenuation members with respect to the particle beam (50) in an initial condition for instance. The left side of the rightmost part of the second energy attenuation member (B) is vertically aligned with the left side of the first energy attenuation member (A). The drive unit (10) is configured for moving simultaneously the first energy attenuation member (A) at a first speed (VA1) and the second energy attenuation member (B) at a second speed (VB1) across the charged particle beam (50) during a first movement. Specific here is that, during said first movement, the horizontal (X) component of the instantaneous first speed (VA1) is equal to the horizontal (X) component of the instantaneous second speed (VB1). This can for example be obtained easily by using a drive unit (10) as shown in Fig.3 and by blocking the first motor (M1) at standstill during the first movement, so that the first energy attenuation member (A) does not move with respect to the second energy attenuation member (B).
Thanks to the special cross sections of the first and the second energy attenuation members as described hereinabove (see parts P1+P1' and P2) and thanks to their equal instantaneous speeds in the X direction, it will be easily understood that a total distance travelled by the particles of the particle beam (50) through the first and the second energy attenuation members - and therefore a total energy attenuation of these particles by the energy degrader - remains constant during the first movement.

Fig. 5b shows the positions of the first and the second energy attenuation members with respect to the particle beam (50) at an end of the first movement, namely when the first and the second energy attenuation members have moved over a horizontal (X) distance which is preferably a little smaller than Dx1 so that the particle beam (50) still crosses both the first and the second energy attenuation members at the end of the first movement.

The drive unit (10) is further configured for then moving the first energy attenuation member (A) at a third speed (VA2) across the charged particle beam (50) during a second movement, the average third speed (VA2) over the second movement being larger than the average second speed (VB1) over the first movement. In this example, the second movement is opposite in direction to the first movement.

Fig. 5c shows the positions of the first and the second energy attenuation members with respect to the particle beam (50) at an end of the second movement, namely when the first energy attenuation member (A) has moved to the left over a horizontal (X) distance of Dx1, so that the first energy attenuation member (A) is again positioned in front of the mating part of the second energy attenuation member (B). At this point in time, a total distance travelled by the particle beam (50) through the first and the second energy attenuation members - and therefore a total energy attenuation of the particle beam (50) - will be larger than during the first movement (Fig. 5a and 5b).

From this point in time, one may repeat a sequence of the first and second movements described in relation to Figs. 5a and 5b, as many times as necessary, provided one does not exceed the total width (X) of the second energy attenuation member (B) of course.

This configuration is particularly useful for particle therapy systems, where a target (200), such as a tumour for example, is to be irradiated layer by layer with a particle beam (50), these layers being at different depths into the body of the patient. In such cases, it is desirable to be able to change the energy of the particle beam (50) very quickly and/or very accurately when the system passes from one layer to another layer. The configuration of Figs 5a, 5b and 5c permits to achieve this in the following way. First, the energy degrader is positioned with respect to the particle beam (50) as shown in Fig.5a. The particle beam (50) is then turned on to irradiate a first layer of the tumour (e.g. the deepest layer). While irradiating said layer, the drive unit (10) performs the first movement of the first and the second energy attenuation members at a relatively low speed (VA1x = VA2x). As explained, the total energy attenuation - and therefore the energy of the beam at the exit of the energy degrader - remains constant during this first movement. At the end of the first movement (Fig.5b), the beam is preferably turned off. While the beam is off, the drive unit (10) performs the second movement of the first energy attenuation member (A) at a relatively high speed (VA2). At the end of the second movement (Fig.5c), the beam is turned on again. At this point in time, the total energy attenuation is higher -and therefore the energy of the beam at the exit of the energy degrader is lower than in the course of the first movement.Another layer of the tumour, less deep than the first layer, can then start to be irradiated. Since the mass (m_{A}) of the first energy attenuation member (A) is smaller than the mass (m_{B}) of the second energy attenuation member (B), the second movement can be made very quickly and with high accuracy. This reduces the time needed for changing the beam energy between two layers, which reduces the treatment time.

It will be obvious that one may also proceed the other way around, i.e. by initially positioning the first energy attenuation member (A) right above the matching portion of the thickest part of the second energy attenuation member (B) (left side in Fig. 5a) and to move the first and second energy attenuation member to the left during the first movement, and then to move the first energy attenuation member (A) to the right during the second movement.

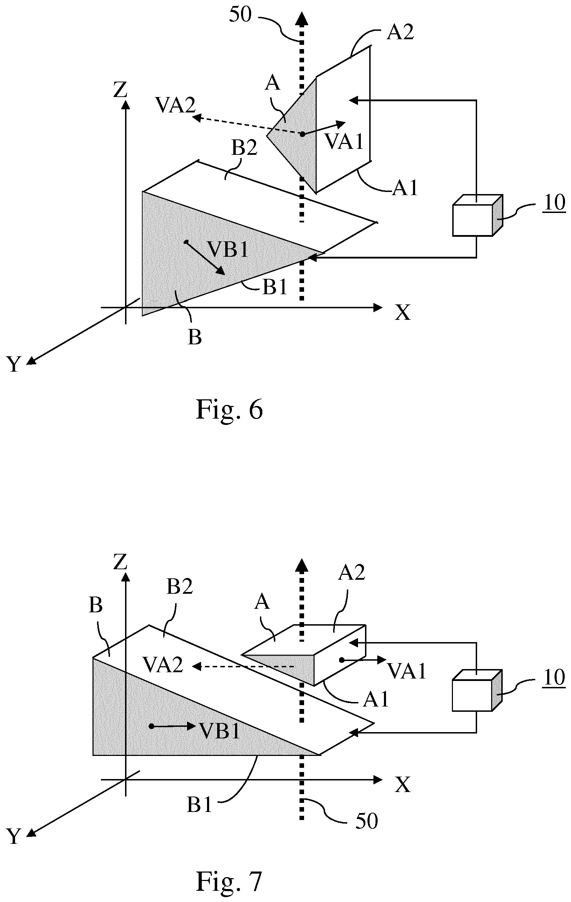

Fig.6 schematically shows a 3D view a more preferred energy degrader according to the invention. It is identical to the ones described hereinabove except that the first energy attenuation member (A) has the shape of a wedge presenting a first beam entry face (A1) and a an opposed first beam exit face (A2), and the second energy attenuation member (B) has the shape of a wedge presenting a second beam entry face (B1) and an opposed second beam exit face (B2). The first and second beam entry faces are flat faces. The first and second beam exit faces are also flat faces. Furthermore, the dive unit (10) is configured for moving the first and second energy attenuation member in translation across the particle beam (50). Beyond the fact that they are easier to design and to manufacture, the advantage of wedge shaped attenuation members is that the amount of energy attenuation can be more freely and more accurately varied following to the second movement. In case the slopes of the two wedges are not the same, it will be obvious that the first and the second speed must be different during the first movement in order to keep the total energy attenuation constant.

Fig.7 schematically shows a 3D view of an even more preferred energy degrader according to the invention. It is identical to the one of Fig.6, except that the first beam entry face (A1) is parallel to the second beam exit face (B2), the first beam exit face (A2) is parallel to the second beam entry face (B1), and that - during the first movement - the horizontal (X) component of the instantaneous first speed (VA1) is equal to the horizontal (X) component of the instantaneous second speed (VB1). This can for example be obtained easily by using a drive unit (10) as shown in Fig.3 and by blocking the first motor (M1) at standstill during the first movement, so that the first energy attenuation member
(A) does not move with respect to the second energy attenuation member (B) during the first movement.

In such a configuration, it will be easily understood that a total distance travelled by the particle beam (50) through the first and the second energy attenuation members - and therefore a total energy attenuation of the particle beam (50) by the energy degrader - remains constant during the first movement, and that the total energy attenuation of the particle beam (50) quickly varies following to the second movement.

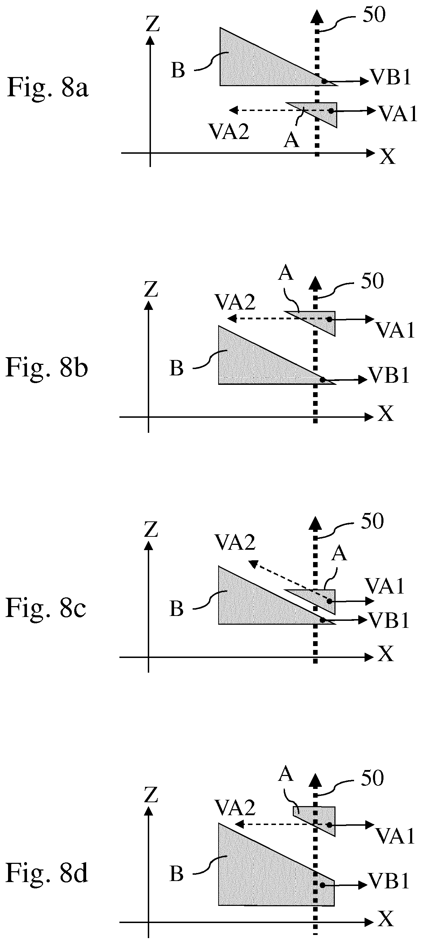

Figs. 8a, 8b, 8c and 8d schematically show cross sections according to a plane parallel to the XZ plane of the energy degrader of Fig. 7 and according to various arrangements of the first and second energy attenuation member and of their respective movements. For the sake of clarity, the drive unit (10), although present, is not shown.

Figs. 8b and 8c are more preferred arrangements since the beam enters and exits the energy degrader perpendicularly to its entry and exit surfaces.
Fig. 8c is an even more preferred arrangement since it allows keeping a smaller gap between the two wedges than in the case of Fig.8b.
Fig. 8d illustrates the case where the wedges are truncated at one of their ends. As a general remark, it is to be noted that the lateral faces (the faces facing the YZ plane) of the wedges are preferably flat and parallel to each other, although this is not mandatory.

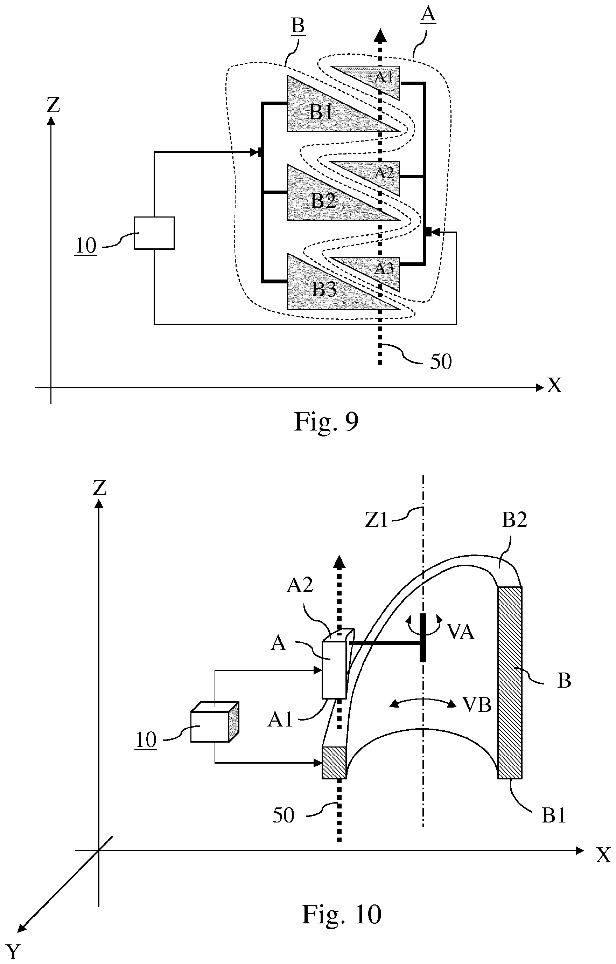

Fig.9 schematically shows a variant of an energy degrader according to an example. It is identical to the energy degraders described hereinabove, except that the first energy attenuation member (A) is subdivided into a plurality of mechanically interconnected first sub-members (Ai), and that the second energy attenuation member (B) is subdivided into a plurality of mechanically interconnected second sub-members (Bi). In this example wedge-shaped sub-members are shown, but any other shape may used as well.

When they have the shape of a wedge with mutually parallel beam entry and exit faces as shown in Figs. 7 to 8, the energy attenuation members may for example have the following characteristics, particularly in the framework of a particle therapy system wherein the beam energy must be attenuated, from for example 230 MeV at the input of the degrader, to an energy ranging between 50MeV and 230 MeV at the output of the degrader:

| | Second attenuation member (B) | First attenuation member (A) |
|---|---|---|
| Width in X direction [cm] | 64 | 8 |
| Height in Z direction [cm] | 16 | 2 |
| Angle of wedge [°] | 14.04 | 14.04 |
| Depth in Y direction [cm] | 2 | 2 |
| Volume [cm3] | 1024 | 16 |
| Density [N/A] | 1.85 | 1.85 |
| Mass [g] | 1894.4 | 29.6 |

The first and second energy attenuation members as described hereinabove may also be fold around a central axis (Z1) which is preferably parallel the particle beam (50) at the location along the beam path where the particle beam (50) crosses the energy degrader. In such a case, the drive unit (10) is configured for driving the first (A) and second (B) energy attenuation members into rotation around the central axis (Z1) and according to the respectively described speeds (VA1, VB1, VA2), which are of course rotational speeds in this case.

Fig.10 schematically shows an exemplary and preferred energy degrader which may be obtained this way as well as its position and orientation with respect to the particle beam (50). In this example, the first energy attenuation member (A) presents a first beam entry face (A1) having the shape of a portion of a first helicoidal ramp and a an opposed first beam exit face (A2) having the shape of a portion of a flat ring. The second energy attenuation member (B) presents a second beam entry face (B1) having the shape of a portion of a flat ring and an opposed second beam exit face (B2) having the shape of a second helicoidal ramp. The first helicoidal ramp is coaxial with the second helicoidal ramp. Preferably, the first helicoidal ramp is matching with the second helicoidal ramp, the first beam exit face (A2) is parallel to the second beam entry face (B1), and - during the first movement - the instantaneous first rotational speed (VA1) is equal to the instantaneous second rotational speed (VB1). This preferred configuration is in fact a rotational equivalent of the configuration of Fig.7.

In any of the configurations described hereinabove, the drive unit (10) and the first and second attenuation members are preferably configured in such a way that a maximum gap between the first and second attenuation members in the course of the first and the second movements is smaller than 5 cm, preferably smaller than 1 cm, preferably smaller than 100mm, preferably smaller than 10mm, preferably smaller than 1 mm.

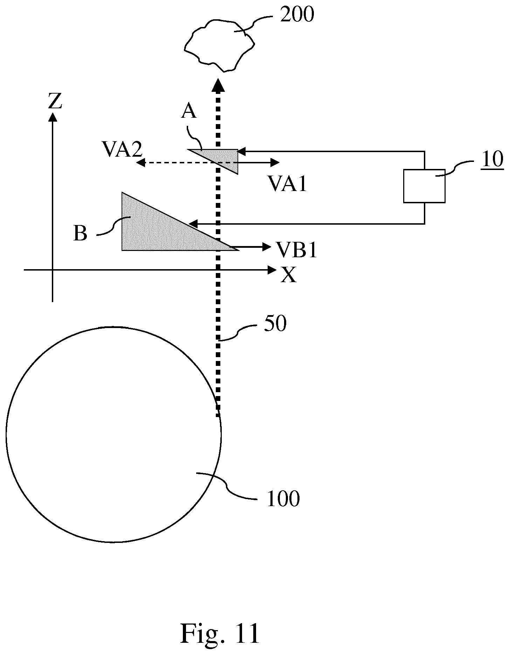

As schematically shown on Fig.11, the disclosure also concerns a particle therapy system configured for irradiating a target (200) with a charged particle beam (50). Said particle therapy system comprises a particle accelerator (100) configured for outputting a charged particle beam (50), such as a beam of protons or carbon ions for example, and an energy degrader as described hereinabove for attenuating the energy of said charged particle beam (50) before it reaches the target (200). In the example of Fig 11, the first and second energy attenuation members of the energy degrader are wedge-shaped, but any other shape as described hereinabove may be used as well. Preferably, the particle accelerator (100) is a fixed-energy accelerator. More preferably, the particle accelerator (100) is a cyclotron, for example a synchrocyclotron.

The present invention has been described in terms of specific embodiments, which are illustrative of the invention and not to be construed as limiting. More generally, it will be appreciated by persons skilled in the art that the present invention is not limited by what has been particularly shown and/or described hereinabove.
Reference numerals in the claims do not limit their protective scope.
Use of the verbs "to comprise", "to include", "to be composed of", or any other variant, as well as their respective conjugations, does not exclude the presence of elements other than those stated.
Use of the article "a", "an" or "the" preceding an element does not exclude the presence of a plurality of such elements.

The invention is defined by the appended claims.

## Claims

1. An energy degrader for attenuating the energy of a charged particle beam (50), said energy degrader comprising :
- a first energy attenuation member (A) adapted to attenuate the energy of charged particles crossing said first attenuation member,
- a second energy attenuation member (B) adapted to attenuate the energy of charged particles crossing said second attenuation member,
- a drive unit (10) operably connected to the first and to the second energy attenuation member and configured for moving the first and/or the second energy attenuation member across the charged particle beam (50),
- the mass (m_{A}) of the first energy attenuation member (A) is smaller than the mass (m_{B}) of the second energy attenuation member (B),
- the drive unit (10) is configured for moving the first energy attenuation member (A) at a first speed (VA1) and the second energy attenuation member (B) at a second speed (VB1) across the charged particle beam (50) during a first movement,
- the drive unit (10) is configured for moving the first energy attenuation member (A) at a third speed (VA2) across the charged particle beam (50) during a second movement, and
- the average third speed (VA2) over the second movement is larger than the average second speed (VB1) over the first movement,
**characterised in that**
- the drive unit is configured for moving simultaneously the first and the second energy attenuation member across the charged particle beam during the first movement and
- the shapes of the first and second energy attenuation members are such that a total energy attenuation of the particle beam remains constant during the first movement.

2. An energy degrader according to claim 1, **characterized in that** the mass (m_{A}) of the first energy attenuation member (A) is smaller than 0.5 times the mass (m_{B}) of the second energy attenuation member (B).

3. An energy degrader according to any of previous claims, **characterized in that** the average third speed (VA2) over the second movement is larger than two times the average second speed (VB1) over the first movement.

4. An energy degrader according to any of previous claims, **characterized in that**, during the first movement, the instantaneous first speed (VA1) is equal to the instantaneous second speed (VB1).

5. An energy degrader according to any of previous claims, **characterized in that** the drive unit (10) comprises:
- a first motor (M1) configured for moving the first energy attenuation member (A) at the first speed during the first movement and at the third speed during the second movement, and
- a second motor (M2) configured for moving the second energy attenuation member (B) at the second speed during the first movement.

6. An energy degrader according to claim 5, **characterized in that** the stator of the first motor (M1) is rigidly connected to the second energy attenuation member (B).

7. An energy degrader according to any of previous claims, **characterized in that**:
- the first energy attenuation member (A) has the shape of a wedge presenting a first beam entry face (A1) and an opposed first beam exit face (A2),
- the second energy attenuation member (B) has the shape of a wedge presenting a second beam entry face (B1) and an opposed second beam exit face (B2),
- said first and second beam entry faces being flat faces,
- said first and second beam exit faces being flat faces,
- the first and the second movements are translational movements across the particle beam (50).

8. An energy degrader according to claim 7, **characterized in that**:
- the fist beam entry face (A1) is parallel to the second beam exit face (B2),
- the fist beam exit face (A2) is parallel to the second beam entry face (B1),
- during the first movement, the instantaneous first speed (VA1) is equal to the instantaneous second speed (VB1).

9. An energy degrader according to any of claims 1 to 6, **characterized in that**:
- the first energy attenuation member (A) presents a fist beam entry face (A1) having the shape of a portion of a first helicoidal ramp and an opposed fist beam exit face (A2) having the shape of a portion of a flat ring,
- the second energy attenuation member (B) presents a second beam entry face (B1) having the shape of a portion of a flat ring and an opposed second beam exit face (B2) having the shape of a second helicoidal ramp,
- the first helicoidal ramp is coaxial with the second helicoidal ramp, and
- the first and the second movements are rotational movements around a central axis (Z1).

10. An energy degrader according to claim 9, **characterized in that**:
- the first helicoidal ramp is matching with the second helicoidal ramp,
- the fist beam exit face (A2) is parallel to the second beam entry face (B1), and
- during the first movement, the instantaneous first rotational speed (VA1) is equal to the instantaneous second rotational speed (VB1).

11. An energy degrader according to any of previous claims, **characterized in that** the drive unit (10) and the first and second attenuation members are configured in such a way that a maximum gap between the first and second attenuation members in the course of the first and the second movements is smaller than 1 cm.

12. A particle therapy system comprising a particle accelerator (100) configured for producing a charged particle beam (50), and comprising an energy degrader according to any of previous claims for attenuating the energy of said charged particle beam (50).

13. A particle therapy system according to claim 12, **characterized in that** the particle accelerator (100) is a fixed-energy accelerator.

14. A particle therapy system according to claim 12 or 13, **characterized in that** the particle accelerator (100) is a cyclotron.

15. A particle therapy system according to claim 14, **characterized in that** the particle accelerator (100) is a synchrocyclotron.

## Patentansprüche

1. Energieabschwächer zur Abschwächung der Energie eines geladenen Partikelstrahls (50), wobei der genannte Energieabschwächer umfasst:
- ein erstes Energieabschwächungsglied (A), das zur Abschwächung der Energie von geladenen Partikeln geeignet ist, die das genannte erste Abschwächungsglied durchkreuzen,
- zweites Energieabschwächungsglied (B), das zur Abschwächung der Energie von geladenen Partikeln geeignet ist, die das genannte zweite Abschwächungsglied kreuzen,
- Antriebsgerät (10), das betriebsbereit an das erste und an das zweite Energieabschwächungsglied angeschlossen und konfiguriert ist, um das erste und / oder das zweite Energieabschwächungsglied durch den geladenen Partikelstrahl (50) zu bewegen,
- die Masse (m_{A}) des ersten Energieabschwächungsgliedes (A) ist kleiner als die Masse (m_{B}) des zweiten Energieabschwächungsgliedes (B),
- die Antriebseinheit (10) ist zum Bewegen des ersten Energieabschwächungsgliedes (A) bei einer ersten Geschwindigkeit (VA1) und das zweite Energieabschwächungsglied (B) in einer zweiten Geschwindigkeit (VB1) durch den geladenen Partikelstrahl (50) während einer ersten Bewegung konfiguriert,
- die Antriebseinheit (10) ist zum Bewegen des ersten Energieabschwächungsgliedes (A) bei einer dritten Geschwindigkeit (VA2) durch den geladenen Partikelstrahl (50) während einer zweiten Bewegung konfiguriert und
- die durchschnittliche dritte Geschwindigkeit (VA2) über der zweiten Bewegung ist größer als die durchschnittliche Geschwindigkeit (VB1) über der ersten Bewegung,
**dadurch gekennzeichnet, dass**
- das Antriebsgerät zur gleichzeitigen Bewegung des ersten und des zweiten Energieabschwächungsgliedes durch den geladenen Partikelstrahl während der ersten Bewegung konfiguriert ist und
- die Formen des ersten und des zweiten Energieabschwächungsgliedes derart sind, dass eine totale Energieabschwächung des Partikelstrahls während der ersten Bewegung konstant bleibt.

2. Energieabschwächer gemäß Anspruch 1, **dadurch gekennzeichnet, dass** die Masse (m_{A}) des ersten Energieabschwächungsgliedes (A) kleiner ist als das 0,5-fache der Masse (m_{B}) des zweiten Energieabschwächungsgliedes (B).

3. Energieabschwächer gemäß einem der voranstehenden Ansprüche, **dadurch gekennzeichnet, dass** die durchschnittliche dritte Geschwindigkeit (VA2) über der zweiten Bewegung größer ist als das Zweifache der durchschnittlichen zweiten Geschwindigkeit (VB1) über der ersten Bewegung.

4. Energieabschwächer gemäß einem der voranstehenden Ansprüche, **dadurch gekennzeichnet, dass** während der ersten Bewegung die sofortige erste Geschwindigkeit (VA1) gleich der sofortigen zweiten Geschwindigkeit (VB1) ist.

5. Energieabschwächer gemäß irgendeinem der voranstehenden Ansprüche, **dadurch gekennzeichnet, dass** das Antriebsgerät (10) umfasst:
- einen ersten Motor (M1), der zum Bewegen des ersten Energieabschwächungsgliedes (A) bei der ersten Geschwindigkeit während der ersten Bewegung und bei der dritten Geschwindigkeit während der zweiten Bewegung konfiguriert ist, und
- einen zweiten Motor (M2), der zum Bewegen des zweiten Energieabschwächungsgliedes (B) bei der zweiten Geschwindigkeit während der ersten Bewegung konfiguriert ist.

6. Energieabschwächer gemäß Anspruch 5, **dadurch gekennzeichnet, dass** der Stator des ersten Motors (M1) steif an das zweite Energieabschwächungsglied (B) angeschlossen ist.

7. Energieabschwächer gemäß irgendeinem der voranstehenden Ansprüche, **dadurch gekennzeichnet, dass**:
- das erste Energieabschwächungsglied (A) die Form eines Keils hat, der eine erste Strahleingangsseite (A1) und eine entgegengesetzte erste Strahlausgangsseite (A2) aufweist,
- das zweite Energieabschwächungsglied (B) die Form eines Keils hat, der eine zweite Strahleingangsseite (B1) und eine entgegengesetzte zweite Strahlausgangsseite (B2) aufweist,
- wobei die genannte erste und die zweite Eingangsseite flache Seiten sind,
- wobei die genannte erste und die zweite Strahlausgangsseite flache Seiten sind,
- wobei die erste und die zweite Bewegung Parallelverschiebungen durch den Partikelstrahl (50) sind.

8. Energieabschwächer gemäß Anspruch 7, **dadurch gekennzeichnet, dass**:
- die erste Strahleingangsseite (A1) parallel zu der zweiten Strahlausgangsseite (B2) ist,
- die erste Strahlausgangsseite (A2) parallel zu der zweiten Strahleingangsseite (B1) ist,
- die sofortige erste Geschwindigkeit (VA1) während der ersten Bewegung gleich der sofortigen zweiten Geschwindigkeit (VB1) ist.

9. Energieabschwächer gemäß irgendeinem der Ansprüche 1 bis 6, **dadurch gekennzeichnet, dass**:
- das erste Energieabschwächungsglied (A) eine erste Strahleingangsseite (A1) aufweist, die die Form eines Abschnitts einer ersten spiralförmigen Rampe hat, und eine entgegengesetzte erste Strahlausgangsseite (A2), die die Form eines Abschnitts eines flachen Rings hat,
- das zweite Energieabschwächungsglied (B) eine zweite Strahleingangsseite (B1), die die Form eines Abschnitts eines flachen Rings hat, und eine entgegengesetzte zweite Strahlausgangsseite (B2), die die Form einer zweiten spiralförmigen Rampe hat, aufweist
- die erste spiralförmige Rampe koaxial zu der zweiten spiralförmigen Rampe ist und
- die erste und die zweite Bewegung Rotationsbewegungen um eine zentrale Achse (Z1) sind.

10. Energieabschwächer gemäß Anspruch 9, **dadurch gekennzeichnet, dass**:
- die erste spiralförmige Rampe abgestimmt ist auf die zweite spiralförmige Rampe,
- die erste Strahlausgangsseite (A2) parallel zu der zweiten Strahleingangsseite (B1) ist, und
- die sofortige erste Rotationsgeschwindigkeit (VA1) während der ersten Bewegung gleich der sofortigen zweiten Rotationsgeschwindigkeit (VB1) ist.

11. Energieabschwächer gemäß irgendeinem der voranstehenden Ansprüche, **dadurch gekennzeichnet, dass** das Antriebsgerät (10) und das erste und das zweite Abschwächungsglied derart konfiguriert sind, dass eine maximale Lücke zwischen dem ersten und dem zweiten Abschwächungsglied im Verlauf der ersten und der zweiten Bewegung kleiner ist als 1 cm.

12. Partikeltherapiesystem, umfassend einen Partikelbeschleuniger (100), der zur Herstellung eines geladenen Partikelstrahls (50) konfiguriert ist, und umfassend einen Energieabschwächer gemäß irgendeinem der voranstehenden Ansprüche zur Abschwächung der Energie des genannten geladenen Partikelstrahls (50).

13. Partikeltherapiesystem gemäß Anspruch 12, **dadurch gekennzeichnet, dass** der Partikelbeschleuniger (100) ein befestigter Energiebeschleuniger ist.

14. Partikeltherapiesystem gemäß Anspruch 12 oder 13, **dadurch gekennzeichnet, dass** der Partikelbeschleuniger (100) ein Zyklotron ist.

15. Partikeltherapiesystem gemäß Anspruch 14, **dadurch gekennzeichnet, dass** der Partikelbeschleuniger (100) ein Synchrozyklotron ist.

## Revendications

1. Dispositif de dégradation d'énergie pour atténuer l'énergie d'un faisceau de particules chargées (50), ledit dispositif de dégradation d'énergie comprenant:
- un premier élément d'atténuation d'énergie (A) apte à atténuer l'énergie de particules chargées qui croisent ledit premier élément d'atténuation d'énergie,
- un second élément d'atténuation d'énergie (B) apte à atténuer l'énergie des particules chargées qui croisent ledit second élément d'atténuation d'énergie,
- une unité d'entraînement (10) qui est connectée de façon opérationnelle aux premier et second éléments d'atténuation d'énergie et qui est configurée de manière à déplacer le premier et/ou le second élément(s) d'atténuation d'énergie à travers le faisceau de particules chargées (50),
- la masse (m_{A}) du premier élément d'atténuation d'énergie (A) est inférieure à la masse (m_{B}) du second élément d'atténuation d'énergie (B),
- l'unité d'entraînement (10) est configurée de manière à déplacer le premier élément d'atténuation d'énergie (A) à une première vitesse (VA1) et le second élément d'atténuation d'énergie (B) à une seconde vitesse (VB1) à travers le faisceau de particules chargées (50) pendant un premier déplacement,
- l'unité d'entraînement (10) est configurée de manière à déplacer le premier élément d'atténuation d'énergie (A) à une troisième vitesse (VA2) à travers le faisceau de particules chargées (50) pendant un second déplacement, et
- la troisième vitesse moyenne (VA2) lors du premier déplacement est supérieure à la deuxième vitesse moyenne (VB1) lors du premier déplacement,
**caractérisé en ce que**:
- l'unité d'entraînement est configurée de manière à déplacer simultanément les premier et second éléments d'atténuation d'énergie à travers le faisceau de particules chargées pendant le premier déplacement; et
- les formes des premier et second éléments d'atténuation d'énergie sont telles qu'une atténuation d'énergie totale du faisceau de particules reste constante pendant le premier déplacement.

2. Dispositif de dégradation d'énergie selon la revendication 1, **caractérisé en ce que** la masse (m_{A}) du premier élément d'atténuation d'énergie (A) est inférieure à 0,5 fois la masse (m_{B}) du second élément d'atténuation d'énergie (B).

3. Dispositif de dégradation d'énergie selon l'une quelconque des revendications précédentes, **caractérisé en ce que** la troisième vitesse moyenne (VA2) lors du second déplacement est supérieure à deux fois la deuxième vitesse moyenne (VB1) lors du premier déplacement.

4. Dispositif de dégradation d'énergie selon l'une quelconque des revendications précédentes, **caractérisé en ce que**, pendant le premier déplacement, la première vitesse instantanée (VA1) est égale à la deuxième vitesse instantanée (VB1).

5. Dispositif de dégradation d'énergie selon l'une quelconque des revendications précédentes, **caractérisé en ce que** l'unité d'entraînement (10) comprend:
- un premier moteur (M1) configuré de manière à déplacer le premier élément d'atténuation d'énergie (A) à la première vitesse pendant le premier déplacement et à la troisième vitesse pendant le second déplacement; et
- un second moteur (M2) configuré de manière à déplacer le second élément d'atténuation d'énergie (B) à la deuxième vitesse pendant le premier déplacement.

6. Dispositif de dégradation d'énergie selon la revendication 5, **caractérisé en ce que** le stator du premier moteur (M1) est connecté de façon rigide au second élément d'atténuation d'énergie (B).

7. Dispositif de dégradation d'énergie selon l'une quelconque des revendications précédentes, **caractérisé en ce que**:
- le premier élément d'atténuation d'énergie (A) a la forme d'un coin présentant une première face d'entrée de faisceau (A1) et une première face de sortie de faisceau opposée (A2),
- le second élément d'atténuation d'énergie (B) a la forme d'un coin présentant une seconde face d'entrée de faisceau (B1) et une seconde face de sortie de faisceau opposée (B2),
- lesdites première et seconde faces d'entrée de faisceau sont des faces plates,
- lesdites première et seconde faces de sortie de faisceau sont des faces plates,
- les premier et second déplacements sont des déplacement translatifs à travers le faisceau de particules (50).

8. Dispositif de dégradation d'énergie selon la revendication 7, **caractérisé en ce que**:
- la première face d'entrée de faisceau (A1) est parallèle à la seconde face de sortie de faisceau (B2),
- la première face de sortie de faisceau (A2) est parallèle à la seconde face d'entrée de faisceau (B1),
- pendant le premier déplacement, la première vitesse instantanée (VA1) est égale à la deuxième vitesse instantanée (VB1).

9. Dispositif de dégradation d'énergie selon l'une quelconque des revendications 1 à 6, **caractérisé en ce que**:
- le premier élément d'atténuation d'énergie (A) présente une première face d'entrée de faisceau (A1) qui a la forme d'une partie d'une première rampe hélicoïdale et une première face de sortie de faisceau opposée (A2) qui a la forme d'une partie d'un anneau plat,
- le second élément d'atténuation d'énergie (B) présente une seconde face d'entrée de faisceau (B1) qui a la forme d'une partie d'un anneau plat et une seconde partie de sortie de faisceau opposée (B2) qui a la forme d'une seconde rampe hélicoïdale,
- la première rampe hélicoïdale est coaxiale à la seconde rampe hélicoïdale, et
- les premier et second déplacements sont des déplacement rotatifs autour d'un axe central (Z1).

10. Dispositif de dégradation d'énergie selon la revendication 9, **caractérisé en ce que**:
- la première rampe hélicoïdale correspond à la seconde rampe hélicoïdale,
- la première face de sortie de faisceau (A2) est parallèle à la seconde face d'entrée de faisceau (B1), et
- pendant le premier déplacement, la première vitesse de rotation instantanée (VA1) est égale à la deuxième vitesse de rotation instantanée (VB1).

11. Dispositif de dégradation d'énergie selon l'une quelconque des revendications précédentes, **caractérisé en ce que** l'unité d'entraînement (10) et les premier et second éléments d'atténuation d'énergie sont configurés de telle sorte qu'un espace maximum entre les premier et second éléments d'atténuation au cours des premier et second déplacements soit supérieur à 1 cm.

12. Système de hadronthérapie comprenant un accélérateur de particules (100) configuré de manière à produire un faisceau de particules chargées (50), et comprenant un dispositif de dégradation d'énergie selon l'une quelconque des revendications précédentes pour atténuer l'énergie dudit faisceau de particules chargées (50).

13. Système de hadronthérapie selon la revendication 12, **caractérisé en ce que** l'accélérateur de particules (100) est un accélérateur à énergie fixe.

14. Système de hadronthérapie selon la revendication 12 ou 13, **caractérisé en ce que** l'accélérateur de particules (100) est un cyclotron.

15. Système de hadronthérapie selon la revendication 14, **caractérisé en ce que** l'accélérateur de particules (100) est un synchrocyclotron.
